# EUROPEAN PATENT SPECIFICATION

(11) **EP 4 197 475 B1**
(45) Date of publication and mention of the grant of the patent: **01.11.2023**
(21) Application number: 21214965.2
(22) Date of filing: 16.12.2021
(51) Int. Cl.: A61B 34/20, A61B 90/00, G01R 33/28, A61B 6/00, G01R 33/483, A61B 6/12, A61B 90/50

(54) **TECHNIQUE OF DETERMINING A SCAN REGION TO BE IMAGED BY A MEDICAL IMAGE ACQUISITION DEVICE**
TECHNIK ZUR BESTIMMUNG EINER ABTASTREGION, DIE VON EINER VORRICHTUNG ZUR ERFASSUNG MEDIZINISCHER BILDER ABGEBILDET WIRD
TECHNIQUE DE DÉTERMINATION D'UNE RÉGION DE BALAYAGE À IMAGER À L'AIDE D'UN DISPOSITIF D'ACQUISITION D'IMAGES MÉDICALES

(43) Date of publication of application: 21.06.2023
(73) Proprietor: Stryker European Operations Limited, Carrigtwohill, Co. Cork T45 HX08 (IE)
(72) Inventor: HORNECKER, Patrick, 79356 Eichstetten (DE); HERRMANN, Florian, 77963 Schwanau (DE)
(74) Representative: Röthinger, Rainer

(56) References cited:
- US-A1- 2010 041 985
- US-A1- 2014 364 720
- US-A1- 2020 085 511
- US-A1- 2021 169 577
- US-B2- 9 066 673
- US-B2- 9 259 290
- SCHAEFFTER T ET AL: "Automatic scan plane definition for frameless MR-stereotaxy on a clinical scanner using an active surgical device holder", INTERNATIONAL SOCIETY FOR MAGNETIC RESONANCE IN MEDICINE. SCIENTIFIC MEETING AND EXHIBITION. PROCEEDINGS, INTERNATIONAL SOCIETY FOR MAGNETIC RESONANCE IN MEDICINE, US, vol. 3, 22 May 1999 (1999-05-22), page 1961, XP002151958, ISSN: 1524-6965

## Description

### Technical Field

The present disclosure generally relates to medical imaging. In particular, it relates to a method for determining a scan region to be imaged by a medical image acquisition device as well as to an associated apparatus, medical imaging system and computer program product.

### Background

Many clinical procedures rely on medical image data of a patient's body. Such medical image data may comprise computed tomography, CT, image data, magnetic resonance, MR, image data or x-ray image data, for example. The medical image data may be used by doctors to make a diagnosis and may be used by surgeons to plan and verify placement of medical implants in the patient's body.

A further field relying on medical image data is surgical navigation. Surgical navigation usually provides a user (e.g., the surgeon or a surgical robot) with information guiding the user on where to position a tracked surgical instrument or a tracked medical implant relative to the patient's body. Such information may be visualized overlaid with the medical image data of the patient.

All of the above and other clinical procedures require medical image data to be taken of the patient's body or a portion thereof. In some known solutions, areas or volumes to be imaged by a medical image acquisition device for obtaining the medical image data are chosen to be as large as possible. Although this may yield image data representing most or even all portions of the patient's body, the required imaging procedures in these solutions may be time-consuming and, in the case of x-ray based imaging techniques, may lead to a large radiation exposure to staff and the patient.

Document US 9 259 290 B2 discloses systems to facilitate MRI-guided procedures that are configured to generate proximity alerts for an MRI-guided procedure associated with at least one target site and/or at least one avoid zone.

US 2014/364720 A1 discloses a method, a system, and a non-transitory computer readable medium for use in interactive magnetic resonance imaging. An initial region of interest of a subject is scanned to acquire imaging data using an initial imaging protocol. Anatomical labeling information corresponding to a plurality of regions corresponding to the subject may be determined based on the acquired data and/or previously available information. Particularly, determining the anatomical labeling information may include identifying one or more features of interest corresponding to the initial region of interest. Further, input from an operator corresponding to a desired imaging task may be received interactively. The imaging protocol may be updated in real-time by selectively configuring one or more imaging parameters that optimize implementation of the desired imaging task based on the determined anatomical labeling information. A subsequent scan may be performed using the updated imaging protocol for completing the desired imaging task.

Further prior art is known from SCHAEFFTER T ET AL: "Automaic scan plane definition for frameless MR-stereotaxy on a clinical scanner using an active surgical device holder", INTERNATIONAL SOCIETY FOR MAGNETIC RESONANCE IN MEDICINE. SCIENTIFIC MEETING AND EXHIBITION. PROCEEDINGS, INTERNATIONAL SOCIETY FOR MAGNETIC RESONANCE IN MEDICINE, US, vol 3, 22 May 1999, page 1961, ISSN: 1524-6965. As further prior art, reference is made to US 9 066 673 B2, US 2021/169577 A1 and US 2020/085511 A1.

### Summary

There is a need for a technique that solves one or more of the aforementioned or other problems.

According to a first aspect, a method for determining a scan region to be imaged by a medical image acquisition device is provided. The method is performed by at least one processor and comprises obtaining, for each of at least one surgical device, information indicative of one or more poses thereof. The method comprises selecting, based on the one or more poses, at least one anatomical element of a patient's body. The method further comprises determining, based on the selected at least one anatomical element, the scan region to be imaged by the medical image acquisition device.

The method may be a computer-implemented method. The method may in particular be performed by one or more processors of a computer system, including cloud computing resources.

The medical image acquisition device may be a magnetic resonance, MR, imaging device, a computed tomography, CT, imaging device or an x-ray imaging device. The scan region may in some variants be the only region to be imaged by the medical image acquisition device. The scan region may be two-dimensional or three-dimensional. The medical image acquisition device may be configured to acquire or generate two-dimensional or three-dimensional medical image data.

According to the present disclosure, a "pose" may be at least one of a position and an orientation, for example a combination of a position and an orientation. The pose may be represented or defined by (e.g., two or) three spatial coordinates and (e.g., two or) three rotational parameters, for example in the form of a matrix.

The at least one anatomical element may be a (e.g., continuous or homogeneous) volume of the patient's body having at least one property chosen from a predefined physical property and a predefined biological property.

The at least one anatomical element is selected based on previously taken medical image data of at least a portion of the patient's body. The portion comprises the at least one anatomical element.

The at least one anatomical element may be selected from one or more anatomical elements identified in the previously taken medical image data. The one or more anatomical elements may be identified using one or more (e.g., atlas-based) image segmentation or object recognition algorithm(s).

Each pose may be indicative of the respective surgical device having an associated relationship with the at least one anatomical element to be selected, for example touching, extending at least partially into or being aligned with the at least one anatomical element to be selected.

In a first variant, the scan region may be determined such that it includes the selected at least one anatomical element. The scan region may be determined such that it includes a combination of (a) the selected at least one anatomical element and (b) at least one zone surrounding one or more of the selected at least one anatomical element.

In a second variant, the scan region may be determined such that it excludes the selected at least one anatomical element. The scan region may be determined such that it excludes a combination of (a) the selected at least one anatomical element and (b) at least one zone surrounding one or more of the selected at least one anatomical element.

In a third variant, the scan region may be determined such that it includes a first set of the selected at least one anatomical element and excludes a second set of the selected at least one anatomical element. The scan region may be determined such that it includes a combination of (a) the anatomical element(s) of the first set and (b) at least one zone surrounding one or more of the anatomical element(s) of the first set. Alternatively or additionally, the scan region may be determined such that it excludes a combination of (a) the anatomical element(s) of the second set and (b) at least one zone surrounding one or more of the anatomical element(s) of the second set. The first set and the second set may each comprise one or more of the selected at least one anatomical element. The criteria which of the selected anatomical element(s) to include in the first set and which to include in the second set may be predefined or user-defined. Each of the selected at least one anatomical element may be classified into the first set or the second set based on at least one of an associated type, an associated degree of irradiation protection and an associated degree of imaging relevancy of the respective anatomical element. Each of the selected at least one anatomical element may be classified into the first set or the second set based on an associated degree of imaging relevancy or image obstruction of the surgical device whose pose was used for selecting the respective anatomical element(s).

The at least one zone may have a homogeneous or constant and, optionally, predefined thickness.

The scan region may be determined to correspond to, consist of or comprise at least one or exactly one virtual bounding box encasing one or more (e.g., all) of the selected at least one anatomical element. The (e.g., at least one) virtual bounding box may have a predetermined shape. The (e.g., at least one) virtual bounding box may have a predetermined size in exactly (e.g., only) one, exactly two or exactly three spatial dimensions. Each of the at least one virtual bounding box may encase the respective selected anatomical element(s) such that a volume between (i) the virtual bounding box and (ii) the selected anatomical element(s) or the zone surrounding the selected anatomical element(s) fulfils one or more predetermined criteria. The one or more predetermined criteria may comprise at least one of a maximum volumetric size, a maximum extension or distance between the virtual bounding box and the selected anatomical element(s), and a predetermined outer surface shape or size.

The scan region (e.g., each virtual bounding box) may be determined to fulfil one or more constraints chosen from a range of movement of the medical image acquisition device, a spatial imaging range of the medical image acquisition device, a range of movement of a patient support supporting the patient's body or a portion thereof, and a field of view of the medical image acquisition device.

The scan region may be determined such that it comprises a plurality of sub-regions to be imaged separately by the medical image acquisition device. Each of the sub-regions may be determined to fulfil the one or more constraints. Each of the sub-regions may correspond to a different virtual bounding box.

The method may further comprise triggering visualization of an indication of the scan region (e.g., on a display means). The visualization may comprise an indication of one or more of the plurality of sub-regions (e.g., a separate or distinguishable indication for each of the sub-regions).

The method may further comprise obtaining user input indicating a change of the scan region, and updating the scan region based on the user input. Updating the scan region may trigger an update of the visualization of the indication of the scan region.

The method may further comprise instructing the medical image acquisition device to image (e.g., only or exclusively) the scan region. Instructing the medical image acquisition device to image the scan region may trigger movement of a (e.g., the) patient support supporting the patient's body or a portion thereof. Instructing the medical image acquisition device to image the scan region may trigger movement of a (e.g., the) patient support before the imaging. Alternatively or additionally, instructing the medical image acquisition device to image the scan region may trigger movement of a (e.g., the) patient support during the imaging. Instructing the medical image acquisition device to image the scan region may trigger acquisition of separate medical image data (e.g., separate CT image data or separate x-ray image data) for each of the plurality of sub-regions by the medical image acquisition device.

At least one of the one or more poses may be obtained based on planning data indicative of the at least one pose relative to the previously taken medical image data of the portion of the patient's body. In this case, the at least one pose may be defined relative to the previously taken medical image data. The at least one pose may be defined in the coordinate system of the previously taken medical image data. The at least one pose may be a planned pose.

Alternatively or additionally, at least one of the one or more poses may be obtained based on tracking data indicative of the at least one pose relative to the patient's body. In this case, the at least one pose may be defined relative to the patient. The at least one pose may be defined in a coordinate system of a (e.g., surgical) tracking system. The at least one pose may be a tracked pose.

Each of the at least one anatomical element may be a bone such as a vertebra. The at least one surgical device may comprise or be at least one device chosen from (i) a surgical instrument and (ii) a medical implant. The at least one surgical device may comprise different types of surgical instruments. Alternatively or additionally, the at least one surgical device may comprise different types of medical implants.

The method according to the first aspect may be a computer-implemented method. The method of the first aspect may not comprise a step requiring a substantial interaction with the body of a human or an animal. The method of the first step may not imply a substantial health risk, e.g., when carried out with the required care and skill. The method of the first aspect may not comprise a surgical step.

According to a second aspect, an apparatus is provided. The apparatus comprises at least one processor and at least one memory, the at least one memory containing or storing instructions which, when executed by the at least one processor, cause the at least one processor to obtain, for each of at least one surgical device, information indicative of one or more poses thereof, select, based on the one or more poses, at least one anatomical element of a patient's body, and determine, based on the selected at least one anatomical element, a scan region to be imaged by a medical image acquisition device. The at least one memory may contain or store instructions which, when executed by the at least one processor, cause the at least one processor to perform the method according to the first aspect. The apparatus may be configured to perform the method according to the first aspect.

According to a third aspect, a medical imaging system is provided. The medical imaging system comprises the apparatus according to the second aspect and the medical image acquisition device. The medical imaging system may further comprise the patient support described herein. The medical imaging system may further comprise one or more of the at least one surgical device. The medical imaging system may further comprise display means configured to display the visualization. The medical imaging system may further comprise a (e.g., optical or electromagnetic) tracking system configured to generate the tracking data.

According to a fourth aspect, a computer program product is provided. The computer program product comprises program code portions for performing the method according to the first aspect when the computer program product is executed on one or more processors. The computer program product may be stored on one or more (e.g., non-transitory) computer storage media. The computer program product may be carried by a data stream. The computer program product may be stored on the at least one memory of the apparatus according to the second aspect.

### Brief Description of the Drawings

Further details, advantages and aspects of the present disclosure will become apparent from the following embodiments taken in conjunction with the drawings, wherein:
- Fig. 1: shows a medical imaging system in accordance with the present disclosure;
- Fig. 2: shows a method in accordance with the present disclosure; and
- Fig. 3: shows an exemplary scan region in accordance with the present disclosure.

### Detailed Description

In the following description, exemplary embodiments of a medical imaging system, a method, an apparatus and a computer program product will be explained with reference to the drawings. The same reference numerals will be used to denote the same or similar structural features.

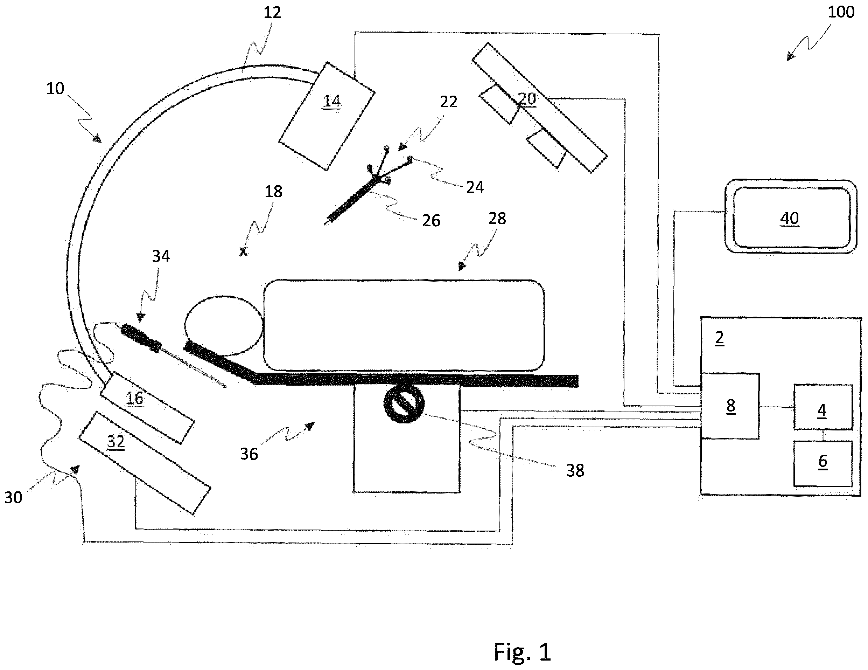

Fig. 1 shows a medical imaging system 100 in accordance with the present disclosure. The imaging system 100 comprises an apparatus 2, wherein the apparatus 2 comprises at least one processor 4 and at least one memory 6. In the shown example, the apparatus 2 further comprises an interface 8.

The medical imaging system 100 further comprises a medical image acquisition device 10. The medical image acquisition device 10 may be a computer tomography, CT, scanner (e.g., comprising a C-arm or a gantry including a through-hole for inserting a patient) or a magnetic resonance, MR, scanner. The medical image acquisition device 10 may be an x-ray apparatus configured for projectional radiography.

In the illustrated example, and for illustration purposes only, the medical image acquisition apparatus 10 is a CT scanner comprising a C-arm 12. An x-ray emitter 14 and an x-ray detector 16 are attached onto opposite ends of the C-arm 12. The C-arm 12 is rotatable around a center point 18. The medical image acquisition device 10 may be communicatively (e.g., wirelessly or via a wired connection) coupled to the apparatus 2 via the interface 8. The medical image acquisition device 10 may be movable (e.g., relative to a patient's body or an operating room) responsive to receipt of a corresponding instruction or trigger signal (e.g., from the apparatus 2). The image acquisition device 10 may be movable at least along a superior-inferior direction of the patient's body, for example during the rotation around the center point 18 (e.g., to generate spiral CT image data).

The medical imaging system 100 may further comprise an optical tracking system 20. The optical tracking system 20 may comprise at least one active sensor chosen from one or more depth sensors, a time-of flight camera and a stereo camera. A signal acquired by the optical tracking system 20 may be transmitted to a processor of the tracking system 20, or to the processor 4 of the apparatus 2. The processor (e.g., the processor 4) may be configured to detect a pose (e.g., at least one of a position and an orientation) of an optical tracker 22. In the illustrated example, the optical tracker 22 comprises four tracking markers 24, although other marker numbers and marker variants are possible.

The optical tracker 22 is attached to a surgical instrument 26. By detecting the pose of the optical tracker 22, the pose of the surgical instrument 26 may be determined based on a known spatial relationship between the tracker 22 and the surgical instrument 26. The pose of the surgical instrument 26 in this example may be determined (e.g., in six degrees of freedom, DOF) in a coordinate system of the optical tracking system 20. Another optical tracker, also referred to as patient tracker, may be fixed relative to the patient's body 28 and localized using the optical tracking system 20. A transformation between a coordinate system of previously taken medical image data of (e.g., a portion of) the patient's body 28 and the pose of the another optical tracker in the coordinate system of the tracking system 20 may be determined. Such a transformation may also be referred to as "registration". This allows determining a pose of the surgical instrument 26 relative to the previously taken medical image data of the patient's body 28.

Alternatively or additionally to the optical tracking system 20, the system 100 may comprise an electromagnetic tracking system 30. The electromagnetic tracking system 30 comprises an electromagnetic field generator 32. A sensor (e.g., comprising a set of detection coils) configured to detect the electromagnetic field may be attached to a surgical instrument 34. A signal acquired by the sensor and representative of the electromagnetic field detected by the sensor may be transmitted to a processor of the tracking system 30, or to the processor 4 of the apparatus 2. The processor (e.g., the processor 4) may be configured to determine the pose of the surgical instrument 34 based on the signal. Another sensor may be attached to a (e.g., the) patient tracker fixed relative to the patient's body and may be localized using the electromagnetic tracking system 30. A transformation between the coordinate system of the previously taken medical image data of (e.g., the portion of) the patient's body 28 and the pose of the patient tracker in the coordinate system of the tracking system 30 may be determined. Again, such a transformation may be referred to as "registration". This allows determining a pose of the surgical instrument 34 relative to the previously taken medical image data of the patient's body 28.

Other tracking systems may be used in addition or alternative to the optical tracking system 20 and the electromagnetic tracking system 30. For example, an ultrasonic tracking system may be used. Numerous techniques of determining a registration between previously taken (e.g., existing, pre-acquired or stored) medical image data of a patient's body on the one hand and the patient's body or the patient tracker on the other hand using a (e.g., optical or electromagnetic) tracking system will be apparent to those skilled in the art.

At least a portion of the body 28 of the patient may be supported by a patient support 36 such as a patient couch. The patient support 36 may be communicatively coupled to the apparatus 2 via the interface 8. The patient support 36 may be configured to move the patient's body upon receipt of a corresponding trigger signal (e.g., from the apparatus 2). The medical image acquisition device 10 may be communicatively coupled (e.g., connected) to the patient support 36 (e.g., via the interface 8 or directly). The patient support 36 may move in synchronization with the medical image acquisition device 10, e.g., by receiving, transmitting or exchanging movement information or movement instructions with the medical image acquisition device 10 via the communicative connection. The patient support 36 may be configured to move the patient's body in three spatial directions. The patient support 36 may be moved by one or more movement motors 38. The one or more movement motors 38 may be arranged in a base 40 of the patient support 36. For example, the patient support 36 may move the patient in the superior-inferior direction while the C-arm 12 rotates around the center point 18. This may result in spiral CT image data. The synchronous or coordinated movement of the patient support 36 and the image acquisition device 10 allows for a vast amount of potential scan regions to be imaged. The shapes and sizes of the potential scan regions may be defined by movement constraints posed by the patient support 36 and the image acquisition device 10.

The apparatus 2 may be communicatively coupled to display means 40. The display means 40 may be configured to display a visualization upon receipt of a corresponding instruction or trigger received from the apparatus 2. The display means 40 may be a stand-alone display screen, an integrated display screen of a navigation system (e.g., comprising the tracking system 20 or the tracking system 30) or a display of an augmented reality device, ARD, such as a head-mounted display, HMD. In another variant, the display means 40 is a projector configured to project the visualization onto a surface (e.g., onto a surface of the patient's body 28).

The medical imaging system 100 comprises the apparatus 2 and the medical image acquisition device 10. The medical imaging system may further comprise the patient support 36. The other components illustrated in Fig. 1 may or may not be part of the medical imaging system 100.

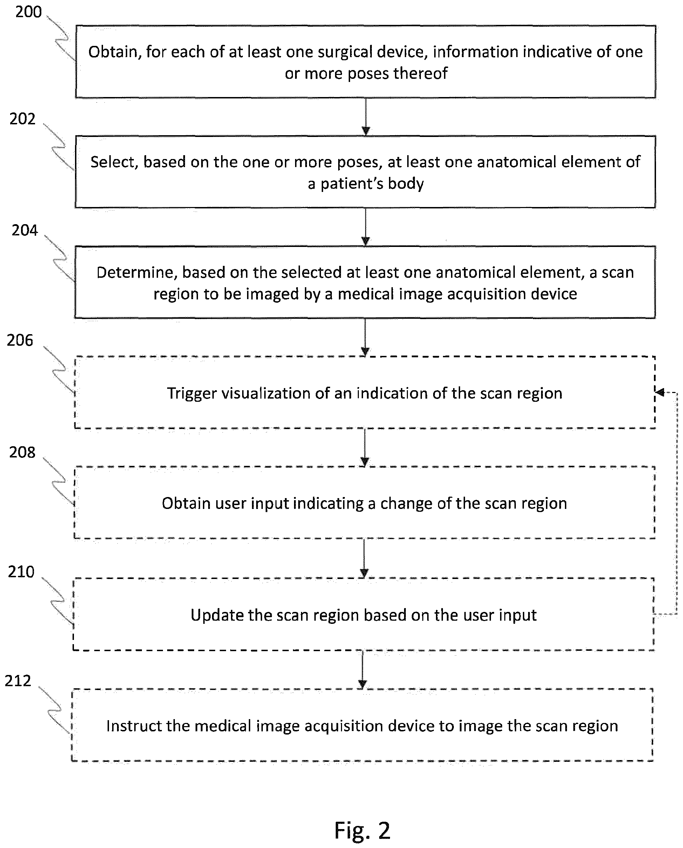

Fig. 2 shows a method in accordance with the present disclosure. The method may be a computer-implemented method. In other words, all of the steps of the method may be performed by one or more processors. The method may be performed by the at least one processor 4 of the apparatus 2. The at least one memory 6 may store instruction which, when executed by the at least one processor 4, cause the at least one processor 4 to perform the method. It is to be noted that the method may be performed by a plurality of (e.g., distributed, remote or cloud) processors. One or more of these processors may be included in the tracking system 20, the tracking system 30, the medical image data acquisition device 10, the patient support 36 or the display means 40.

The method comprises a step 204. In step 204, for each of at least one surgical device, information indicative of one or more poses thereof is obtained. In case of a single surgical device to be considered, information indicative of one or more poses of the single surgical device is obtained. In case of a plurality of surgical devices to be considered, information indicative of one or more poses of each of the plurality of surgical devices is obtained.

The at least one surgical device may comprise a surgical instrument (e.g., the surgical instrument 22 and/or 34). Such a surgical instrument may be a pointer, a chisel, a drill guide, a drill, a k-wire, a screwdriver or the like.

Alternatively or additionally, the at least one surgical device may comprise a medical implant. Such a medical implant may be a surgical screw (e.g., a pedicle screw), an interbody implant (e.g., a spinal cage), a spinal rod, a bone plate or the like.

The at least one surgical device may consist of exactly one surgical instrument or of a plurality of surgical instruments. Alternatively, the at least one surgical device may consist of exactly one medical implant or of a plurality of medical implants.

At least one of the one or more poses may be obtained based on planning data. Such a pose may be referred to as a planned pose. The planning data may be indicative of the at least one (e.g., planned) pose relative to the previously taken medical image data of (e.g., the portion of) the patient's body. The planned pose may be defined in the coordinate system of the previously taken medical image data.

The planning data may be generated based on user input. A planned pose may be determined or defined based on user input indicative of a pose of a (e.g., virtual) surgical device in the previously taken medical image data. For example, a surgeon may define a position and orientation of a (e.g., virtual) pedicle screw in the previously taken medical image data, and this position and orientation may be used as a planned pose indicated by the planning data. As another example, the surgeon may define a trajectory of a drill in a bone represented by the medical image data. In this case, the trajectory defines at least four degrees of freedom of the drill in the coordinate system of the previously taken medical image data as the planned pose.

Alternatively or additionally, the planning data may be determined based on the previously taken medical image data. For example, the previously taken medical image data may be analyzed (e.g., by the processor 4) to detect a surgical device and to determine a pose thereof (e.g., using object recognition), for example in the coordinate system of the previously taken medical image data. The so-determined pose may then be indicated by the planning data and may be referred to as a planned pose.

At least one of the one or more poses may be obtained based on tracking data. Such a pose may be referred to as a tracked pose. The tracking data may be indicative of the at least one (e.g., tracked) pose relative to the patient's body or the patient tracker. The tracking data may be obtained from a tracking system (e.g., the tracking system 20 or the tracking system 30). The tracked pose may be defined in the coordinate system of the tracking system (e.g., the tracking system 20 or the tracking system 30). The tracked pose may be defined relative to a detected pose of the patient tracker and, thus, relative to the patient's body 28. For example, a surgeon may position a screwdriver in a pose relative to the patient's body, which pose he intends to use for inserting a pedicle screw later on or which pose he previously used for inserting the pedicle screw. To acquire the current pose of the positioned screwdriver as the tracked pose, the surgeon may additionally input a user command to the tracking system or the apparatus 2 (e.g., click a button on the apparatus 2, speak a voice command, press a foot pedal or perform a predefined gesture).

To sum up, a difference between a tracked pose and a planned pose may be that the tracked pose may be obtained from the tracking data and may be indicative of a spatial relationship between the surgical device and the patient tracker fixed relative to the patient's body, whereas the planned pose may be obtained from the planning data and may be indicative of a spatial relationship between the previously taken medical image and the (e.g., virtual) surgical device. This allows for at least two workflows for defining the pose(s) obtained in step 202. The surgeon either may plan the pose directly in the previously taken medical image data or may define a tracked pose by positioning the medical device in an intended pose relative to the patient's body. Of course, both workflows may be combined so that the poses obtained in step 202 comprise at least one planned pose and at least one tracked pose.

The method proceeds with a step 204. In step 204, based on the one or more poses obtained in step 200, at least one anatomical element of the patient's body 28 is (e.g., automatically) selected.

The at least one anatomical element is selected based on the previously taken medical image data of at least the portion of the patient's body 28, the portion comprising the at least one anatomical element. The at least one anatomical element may be selected from one or more anatomical elements identified in the previously taken medical image data. The one or more anatomical elements may be identified in the previously taken medical image data using at least one technique chosen from (e.g., atlas-based) image segmentation and object recognition. The at least one anatomical element may have at least one predefined physical property (e.g., a predefined x-ray transparency or a predefined water concentration). The at least one anatomical element may have at least one predefined biological property (e.g., a predefined blood concentration or a predefined tissue type). The at least one anatomical element may be an organ or a bone. For example, in case of CT image data, bones may be identified by segmenting continuous volumes having a Hounsfield value exceeding a predefined CT bone threshold. As another example, in case of T1-weighted MR image data, continuous volumes exhibiting intensity values below a predefined MR bone threshold may be identified as bone surfaces. Other techniques of identifying anatomical elements in medical image data may be used in combination or alternatively thereto. For example, a plurality of vertebrae may be identified in the previously taken medical image data and the at least one anatomical element selected in step 204 may be a vertebra selected from the identified plurality of vertebrae.

Each pose obtained in step 202 may be indicative of the respective surgical device having an associated relationship with the at least one anatomical element to be selected. For example, the pose(s) may be indicative of the respective surgical device touching, extending at least partially into or being aligned with the at least one anatomical element to be selected. For example, the surgeon may set two planned poses by defining a pose for each of two pedicle screws in the previously taken medical image data, such that each of the two pedicle screws extends into a different vertebra. In this case, each of the different vertebrae may be selected in step 204 as the at least one anatomical element. As another example, the surgeon may position a tracked pointer such that a tip of the pointer touches a vertebra of the patient, which vertebra is to be selected. As a still further example, the surgeon may position a tracked drill such that a longitudinal axis of the drill (e.g., a drill axis) is aligned with (e.g., intersects with) a vertebra to be selected.

The method proceeds with a step 206. In step 206, based on the selected at least one anatomical element, a scan region to be imaged by the medical image acquisition device 10 is determined.

In a first variant, the scan region may be determined such that it includes the selected at least one anatomical element. In this variant, the scan region may be determined such that it includes a combination of (a) the selected at least one anatomical element and (b) at least one zone surrounding one or more of the selected at least one anatomical element.

In a second variant, the scan region may be determined such that it excludes the selected at least one anatomical element. In this variant, the scan region may be determined such that it excludes a combination of (a) the selected at least one anatomical element and (b) at least one zone surrounding one or more of the selected at least one anatomical element.

In a third variant, the scan region may be determined such that it includes a first set of the selected at least one anatomical element and excludes a second set of the selected at least one anatomical element. The first set and the second set may be determined based on at least one of an associated type, an associated degree of irradiation protection and an associated degree of imaging relevancy of the respective selected anatomical element(s). For example, an anatomical element may be selected in step 202 and may be associated with the type "vertebra". In case of spinal surgery, image acquisition of vertebrae may be considered important. Thus, the vertebra may be included in the first set and be part of the scan region. The first set and the second set may be determined based on an associated degree of imaging relevancy or image obstruction of the surgical device whose pose was used for selecting the respective anatomical element(s). For example, a bone plate may have a high degree of image obstruction by impeding image quality of body portions in a region surrounding the bone plate due to imaging artifacts (e.g., scattering of x-rays on edges of the bone plate). In this case, a bone onto which the bone plate is attached may be included in the second set and, thus, be excluded from the scan region. The criteria which anatomical element(s) to include in the first set and which to include in the second set may be predefined or user-defined. Also in the third variant, the scan region may be determined such that it includes a combination of (a) the anatomical element(s) of the first set and (b) at least one zone surrounding one or more of the anatomical element(s) of the first set. Alternatively or additionally, the scan region may be determined such that it excludes a combination of (a) the anatomical element(s) of the second set and (b) at least one zone surrounding one or more of the anatomical element(s) of the second set.

The first variant may be selected only if the at least one anatomical element selected in step 204 consists of anatomical element(s) of the first set. The second variant may be selected only if the at least one anatomical element selected in step 204 consists of anatomical element(s) of the second set. The third variant may be selected only if the at least one anatomical element selected in step 204 includes at least one anatomical element of the first set and at least one anatomical element of the second set. Alternatively, which of these three variants is to be used may be predefined or defined by a user. The method may comprise obtaining user input selecting one of the first variant and the second variant, and, optionally, the third variant.

In all three variants, the at least one zone may surround all of the selected anatomical element(s) (e.g., of a same set). The at least one zone may be a virtual layer on the selected anatomical element(s) (e.g., of a same set) and may have a predefined and, optionally, a homogeneous or constant thickness (e.g., a thickness of 0.5mm, 1mm, 1.5mm, 2mm or 5mm). For example, a bone of the patient's body 28 may be selected in step 204, and the scan region may be determined to include not only the selected bone, but also a buffer region or layer (e.g., the zone) of 2mm surrounding the selected bone. This may ensure that the selected bone will be imaged even in case of a slight misalignment of the medical image acquisition device 10 relative to the patient's body 28. As another example, a critical organ (e.g., prone to irradiation damage) of the patient's body 28 may be selected in step 204, and the scan region may be determined to not only exclude the selected organ, but also a safety layer (e.g., the zone) of 5mm surrounding the selected organ. This may ensure that the selected organ will not be irradiated upon image acquisition even in case of a misalignment of the medical image acquisition device 10 relative to the patient's body 28.

The scan region may be determined to correspond to or comprise at least one virtual bounding box encasing one or more (e.g., all) of the selected at least one anatomical element (e.g., of a same set). The at least one virtual bounding box may have a predetermined shape, for example a cylindrical shape. The at least one virtual bounding box may have a predetermined size (e.g., in exactly one, exactly two or exactly three spatial dimensions). For example, the at least one virtual bounding box may have a radius equal to or smaller than a predefined maximal radius. The predetermined shape and/or size may result in the virtual bounding box defining a region or volume of which image data may be taken by the medical image acquisition device 10 in a single image acquisition procedure (e.g., a single scan) or image acquisition step (e.g., a single x-ray shot). For example, the predetermined shape may be a circular cylinder in case of a CT scanner that traverses along one axis while rotating around the same axis during image acquisition. The predetermined shape may correspond to a pyramid or cone in case of a cone-beam x-ray image shot. The at least one virtual bounding box may encase the one or more of the selected at least one anatomical element (e.g., of a same set) such that a volume between the bounding box and the selected at least one anatomical element (e.g., of a same set) fulfils one or more predetermined criteria. The one or more predetermined criteria may comprise at least one of a maximum volumetric size, a maximum extension or distance between (i) the virtual bounding box and (ii) the selected at least one anatomical element (e.g., of a same set) or the at least one zone surrounding the at least one anatomical element (e.g., of the same set). The virtual bounding box may encase both the at least one selected anatomical element (e.g., of a same set) and the at least one zone surrounding the at least one anatomical element (e.g., of the same set).

The scan region may be determined to fulfil one or more constraints chosen from a range of movement of the medical image acquisition device 10, a spatial imaging range of the medical image acquisition device 10 and a range of movement of the patient support 36 supporting the patient's body or a portion thereof. For example, the scan region may be determined to have a maximum extension along a first axis that corresponds to a maximal range of movement of the C-arm 10 along the first axis. Alternatively or additionally, the scan region may be determined to have a maximum extension along a second axis that corresponds to a maximal range of movement of the patient support 36 along the second axis. The scan region may be determined such that the scan region can be imaged with the image acquisition device 10 without repositioning the patient on the patient support 36.

The scan region may be determined such that it comprises a plurality of sub-regions to be imaged separately by the medical image acquisition device 10. The sub-regions may differ from one another in at least one property chosen from size, shape and pose. Each of the sub-regions may be determined to fulfil the one or more constraints. Each of the sub-regions may comprise one or more of the virtual bounding boxes. Each of the sub-regions may consist of one or more of the virtual bounding boxes. In another variant, each of the sub-region may only comprise a portion of a virtual bounding box. Each of the sub-regions may comprise or consist of a separate virtual bounding box, each of which may be determined to fulfil the one or more constraints.

For example, in order to include a plurality of anatomical elements in a single cylindrical scan region, a radius of the cylindrical scan region would need to be chosen such that the most remote of the anatomical elements are still included in the scan region. This may result in a large scan region to be imaged and, thus, increase scanning time and irradiation of the patient's body. To prevent this issue, the sub-regions may be determined such that the sum of the volumes of the sub-regions is lower than the volume of the large scan region. The sub-regions may be determined such that at least one parameter chosen from a scan volume, an image acquisition time and an irradiation of the patient's body 28 is minimized. The scan region may be divided (e.g., as part of step 204) into a plurality of sub-regions to minimize the at least one parameter.

As apparent from Fig. 2, the steps 206, 208, 210 and 212 are considered optional. The method may comprise none, exactly one, exactly two, exactly three or all of these four steps 206, 208, 210, 212. In case the method includes the step 210, it must include the step 208.

In step 206, a visualization of an indication of the scan region is triggered. The visualization may be triggered on the display means 40. The trigger may be sent from the apparatus 2 to the display means 40 via the interface 8. The visualization may comprise an indication of the scan region relative to (e.g., overlaid onto) the previously taken medical image data. The visualization may comprise an indication of one or more of the plurality of sub-regions (e.g., relative to the previously taken medical image data). The visualization may comprise an indication of the virtual bounding box(es). The visualization may comprise an indication of the one or more poses of the at least one surgical device (e.g., the poses obtained in step 202 and used in step 204). The visualization may comprise an indication of the at least one surgical device, for example in the pose(s) obtained in step 202 and used in step 204. The visualization may comprise an indication of the selected at least one anatomical element. The indication(s) may be visualized overlaid over the patient's body, for example by the projector or the HMD.

In step 208, user input indicating a change of the scan region is obtained. For example, a user may change at least one of shape, size and pose of the scan region or of one or more of the sub-regions or of one or more virtual bounding boxes. The user may change the shape, size and/or pose by changing a range of movement of at least one component chosen from the medical image acquisition device 10 and the patient support 36.

In step 210, the scan region is updated based on the user input obtained in step 208. This may involve updating the visualization of the scan region and/or the sub-regions (e.g., by sending another trigger signal to the display means 40).

In step 212, the medical image acquisition device 10 is instructed to image the scan region (e.g., the scan region determined in step 206 or the scan region updated in step 208). Instructing the medical image acquisition device 10 to image the scan region may trigger movement of the patient support 36 supporting the patient's body 28 or the portion thereof. Instructing the medical image acquisition device 10 to image the scan region may trigger movement of the medical image data acquisition device 10 relative to the patient's body 28. Instructing the medical image acquisition device 10 to image the scan region may trigger acquisition of separate medical image data for each of the plurality of sub-regions by the medical image acquisition device 10.

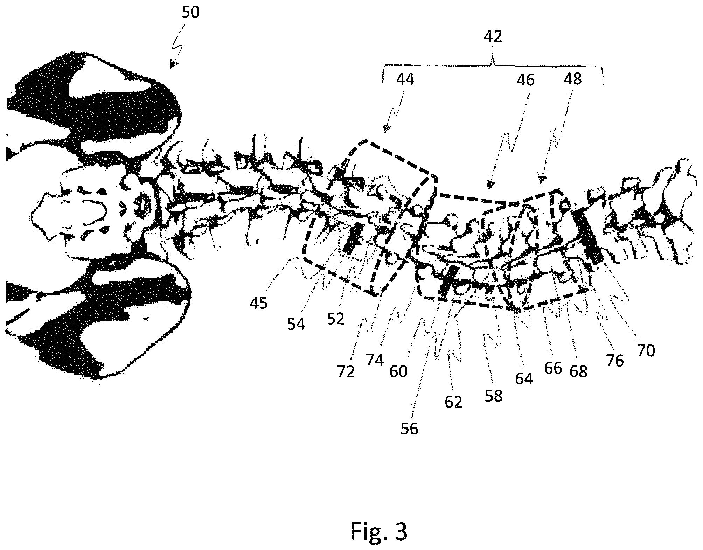

Fig. 3 shows an exemplary scan region 42 in accordance with the present disclosure. The scan region 42 comprises three sub-regions 44, 46, 48. The visualization triggered in step 206 may comprise an indication of the three sub-regions 44, 46, 48 together with a representation (e.g., rendering) 50 of the previously taken medical image data of the patient. The previously taken medical image data may be segmented (e.g., using an atlas-based segmentation) to detect and identify all imaged vertebrae of the patient's body 28.

The sub-region 44 comprises a first vertebra 52. The sub-region 44 is determined such that it comprises not only the first vertebra 52, but also a layer or zone 45 of 2mm surrounding the first vertebra 52. The zone 45 is indicated with a dotted line in Fig. 3. The first vertebra 52 may have been selected in step 204, as the planned pose of pedicle screw 54 in the coordinate system of the (e.g., rendering or representation 50 of the) previously taken medical image data indicates that the pedicle screw 54 extends into the first vertebra 52.

The sub-region 46 comprises a second vertebra 56 and a third vertebra 58. The second vertebra 56 may have been selected in step 204, as the tracked pose of a pedicle screw 60 in the coordinate system of the tracking system 20 relative to the patient's body indicated that the pedicle screw 60 extends into the second vertebra 56. For example, the tracked pose may be transformed from the coordinate system of the tracking system 20 into the coordinate system of the previously taken medical image data (e.g., using a registration as described above) to determine whether the pedicle screw 60 extends into a vertebra, and into which vertebra it extends. The third vertebra 58 may have been selected in step 204, as the tracked pose of a pointer indicated that the pointer was aligned with the third vertebra 58. The pose of the pointer is indicated in Fig. 3 by a dashed line 62 representing a longitudinal axis of the pointer. As can be seen, the line 62 intersects the third vertebra 58, which means that the pointer is considered to be aligned with the vertebra 58. Again, the tracked pose of the pointer may be transformed from the tracking coordinate system into the coordinate system of the previously taken medical image data to determine which of the vertebrae, if any, the pointer was aligned with.

The sub-region 48 comprises a fourth vertebra 64 and a fifth vertebra 66. The sub-region 48 may have been determined in step 206 to exclude a sixth vertebra 68. The sixth vertebra may have been selected in step 204 and classified to fall into the second set of anatomical elements, as a spinal cage 70 is arranged on the vertebra 68 according to the planning data. The arrangement of the spinal cage 70 on the vertebra 68 may be indicated by the planning data (e.g., defined by a user or derived from the previously taken medical image data). The spinal cage 70 may lead to x-ray scattering negatively affecting image quality of a subsequent scan. Thus, the exclusion of the neighboring vertebra 68 from the sub-region 48 and the overall scan region 42 may lead to improved image quality of a subsequent scan. The sub-region 48 may have been determined in step 206 to include two vertebrae 64 and 66 adjacent to the excluded vertebra 68. The vertebrae 64 and 66 may have been selected in step 204 based on two tracked poses indicating a screwdriver touching the vertebrae 64 and 66.

In the example of Fig. 3, each of the sub-regions 44, 46, 48 consists of a different virtual bounding box 72, 74, 76. The shape of each of the bounding boxes 72, 74, 76 in this example is a circular cylinder, as the medical image acquisition device 10 may only be able to image scan regions having such a geometrical shape. The sub-regions 44, 46, 48 may be determined to overlap one another. This may ease image reconstruction (e.g., image stitching) after acquisition of separate medical image data sets for each of the sub-regions 44, 46, 48.

Numerous modifications of the system and method described herein are possible. For example, the sub-regions may be determined such that a first medical image acquisition device takes images of a first set of the sub-regions, wherein a second medical image acquisition device takes images of a second set of the sub-regions. The scan region may be determined for different medical image acquisition devices using the same poses obtained in step 202.

The technique disclosed herein may provide a scan region including relevant anatomical elements. Alternatively or additionally, the scan region may exclude irrelevant or sensitive anatomical elements. The elements to be included or excluded may be easily defined by a user in a planning workflow (e.g., resulting in the planning data) or during navigation (e.g., resulting in the tracking data).

The determined scan region may reduce scanning time and irradiation exposure of the patient and clinical staff. Additional technical effects and advantages will be apparent to those skilled in the art in view of the technique disclosed herein.

## Claims

1. A method for determining a scan region (42) to be imaged by a medical image acquisition device (10), the method being performed by at least one processor (4) and comprising:
obtaining (202), for each of at least one surgical device (26; 34; 54; 60; 70), information indicative of one or more poses thereof;
selecting, based on the one or more poses, at least one anatomical element (52; 56; 58; 64; 66; 68) of a patient's body; and
determining, based on the selected at least one anatomical element (52; 56; 58; 64; 66; 68), the scan region (42) to be imaged by the medical image acquisition device (10),
wherein the at least one anatomical element (52; 56; 58; 64; 66; 68) is selected based on previously taken medical image data of at least a portion of the patient's body (28), the portion comprising the at least one anatomical element (52; 56; 58; 64; 66; 68).

2. The method of claim 1,
wherein the at least one anatomical element (52; 56; 58; 64; 66; 68) is selected from one or more anatomical elements (52; 56; 58; 64; 66; 68) identified in the previously taken medical image data.

3. The method of claim 1 or 2,
wherein each pose is indicative of the respective surgical device (26; 34; 54; 60; 70) having an associated relationship with the at least one anatomical element (52; 56; 58; 64; 66; 68) to be selected, for example touching, extending at least partially into or being aligned with the at least one anatomical element (52; 56; 58; 64; 66; 68) to be selected.

4. The method of any one of claims 1 to 3,
wherein the scan region (42) is determined such that it includes or excludes the selected at least one anatomical element (52; 56; 58; 64; 66; 68) or such that it includes a first set of the selected at least one anatomical element (52; 56; 58; 64; 66) and excludes a second set of the selected at least one anatomical element (68).

5. The method of claim 4,
wherein the scan region (42) is determined to correspond to or comprise at least one virtual bounding box (72, 74, 76) encasing one or more of the selected at least one anatomical element (52; 56; 58; 64; 66).

6. The method of any one of claims 1 to 5,
wherein the scan region (42) is determined to fulfil one or more constraints chosen from
(i) a range of movement of the medical image acquisition device (10),
(ii) a spatial imaging range of the medical image acquisition device (10), and
(ii) a range of movement of a patient support (36) supporting the patient's body (28) or a portion thereof.

7. The method of any one of claims 1 to 6,
wherein the scan region (42) is determined such that it comprises a plurality of sub-regions (44; 46; 48) to be imaged separately by the medical image acquisition device (10).

8. The method of any one of claims 1 to 7, further comprising:
triggering (206) visualization of an indication of the scan region (24).

9. The method of any one of claims 1 to 8, further comprising:
obtaining (208) user input indicating a change of the scan region (42); and
updating (210) the scan region (42) based on the user input.

10. The method of any one of claims 1 to 9, further comprising:
instructing (212) the medical image acquisition device (10) to image the scan region (24).

11. The method of claim 10,
wherein instructing (212) the medical image acquisition device (10) to image the scan region (24) triggers movement of a patient support (36) supporting the patient's body (28) or a portion thereof.

12. The method of claim 10 or 11 as far as depending on claim 7,
wherein instructing (212) the medical image acquisition device (10) to image the scan region (24) triggers acquisition of separate medical image data for each of the plurality of sub-regions (44; 46; 48) by the medical image acquisition device (10).

13. The method of any one of claims 1 to 12,
wherein at least one of the one or more poses is obtained based on planning data indicative of the at least one pose relative to the previously taken medical image data of the portion of the patient's body (28).

14. The method of any one of claims 1 to 13,
wherein at least one of the one or more poses is obtained based on tracking data indicative of the at least one pose relative to the patient's body (28).

15. The method of any one of claims 1 to 14,
wherein each of the at least one anatomical element (52; 56; 58; 64; 66; 68) is a bone, and
wherein the at least one surgical device (26; 34; 54; 60; 70) comprises at least one device chosen from (i) a surgical instrument (26; 34) and (ii) a medical implant (54; 60; 70).

16. An apparatus (2) comprising at least one processor (4) and at least one memory (6), the at least one memory (6) containing instructions which, when executed by the at least one processor (4), cause the at least one processor to:
obtain (202), for each of at least one surgical device (26; 34; 54; 60; 70), information indicative of one or more poses thereof;
select (204), based on the one or more poses, at least one anatomical element (52; 56; 58; 64; 66; 68) of a patient's body; and
determine (206), based on the selected at least one anatomical element (52; 56; 58; 64; 66; 68), a scan region (42) to be imaged by a medical image acquisition device (10),
wherein the at least one anatomical element (52; 56; 58; 64; 66; 68) is selected based on previously taken medical image data of at least a portion of the patient's body (28), the portion comprising the at least one anatomical element (52; 56; 58; 64; 66; 68).

17. The apparatus (2) of claim 16, configured to perform the method of any one of claims 2 to 15.

18. A medical imaging system (100) comprising the apparatus (2) of claim 16 and the medical image acquisition device (10), and, optionally, a patient support supporting the patient's body (28) or a portion thereof.

19. A computer program product comprising program code portions for performing the method of any one of claims 1 to 15 when the computer program product is executed on one or more processors (4).

## Patentansprüche

1. Verfahren zum Bestimmen eines Erfassungsbereichs (42), der von einer medizinischen Bilderfassungsvorrichtung (10) abzubilden ist, wobei das Verfahren von mindestens einem Prozessor (4) durchgeführt wird und umfasst:
Erhalten (202) von Informationen für jedes von mindestens einem chirurgischen Gerät (26; 34; 54; 60; 70), die eine oder mehrere Posen desselben anzeigen;
Auswählen mindestens eines anatomischen Elements (52; 56; 58; 64; 66; 68) des Körpers eines Patienten auf Grundlage der einen oder mehreren Posen; und
Bestimmen, auf Grundlage des ausgewählten mindestens einen anatomischen Elements (52; 56; 58; 64; 66; 68), des Erfassungsbereichs (42), der von der medizinischen Bilderfassungsvorrichtung (10) abzubilden ist,
wobei das mindestens eine anatomische Element (52; 56; 58; 64; 66; 68) auf Grundlage von zuvor aufgenommenen medizinischen Bilddaten von mindestens einem Teil des Körpers (28) des Patienten ausgewählt wird, wobei der Teil das mindestens eine anatomische Element (52; 56; 58; 64; 66; 68) umfasst.

2. Verfahren nach Anspruch 1,
wobei das mindestens eine anatomische Element (52; 56; 58; 64; 66; 68) aus einem oder mehreren anatomischen Elementen (52; 56; 58; 64; 66; 68) ausgewählt wird, die in den zuvor aufgenommenen medizinischen Bilddaten identifiziert wurden.

3. das Verfahren nach Anspruch 1 oder 2,
wobei jede Pose anzeigt, dass das jeweilige chirurgische Gerät (26; 34; 54; 60; 70) eine zugeordnete Beziehung zu dem mindestens einen auszuwählenden anatomischen Element (52; 56; 58; 64; 66; 68) hat, beispielsweise das mindestens eine auszuwählende anatomische Element (52; 56; 58; 64; 66; 68) berührt, sich zumindest teilweise in dieses hinein erstreckt oder auf dieses ausgerichtet ist.

4. das Verfahren nach einem der Ansprüche 1 bis 3,
wobei der Erfassungsbereich (42) so bestimmt wird, dass er das ausgewählte mindestens eine anatomische Element (52; 56; 58; 64; 66; 68) einschließt oder ausschließt, oder so, dass er einen ersten Satz des ausgewählten mindestens einen anatomischen Elements (52; 56; 58; 64; 66) einschließt und einen zweiten Satz des ausgewählten mindestens einen anatomischen Elements (68) ausschließt.

5. Verfahren nach Anspruch 4,
wobei der Erfassungsbereich (42) so bestimmt wird, dass er mindestens einer virtuellen Begrenzung-Box (72, 74, 76) entspricht oder diese umfasst, welche ein oder mehrere des ausgewählten mindestens einen anatomischen Elements (52; 56; 58; 64; 66) umschließt.

6. Verfahren nach einem der Ansprüche 1 bis 5,
wobei der Erfassungsbereich (42) so bestimmt wird, dass er eine oder mehrere Kriterien erfüllt, die ausgewählt sind aus:
(i) einem Bewegungsbereich der medizinischen Bilderfassungsvorrichtung (10),
(ii) einem räumlichen Erfassungsbereich der medizinischen Bilderfassungsvorrichtung (10), und
(ii) einem Bewegungsbereich einer Patientenliege (36), die den Körper (28) des Patienten oder einen Teil davon trägt.

7. Verfahren nach einem der Ansprüche 1 bis 6,
wobei der Erfassungsbereich (42) so bestimmt wird, dass er eine Vielzahl von Unterbereichen (44; 46; 48) umfasst, die von der medizinischen Bilderfassungsvorrichtung (10) getrennt abzubilden sind.

8. Verfahren nach einem der Ansprüche 1 bis 7, ferner umfassend:
Auslösen (206) einer Visualisierung einer Indikation des Erfassungsbereich (24).

9. Verfahren nach einem der Ansprüche 1 bis 8, ferner umfassend:
Erhalten (208) einer Benutzereingabe, die eine Änderung des Erfassungsbereichs (42) anzeigt; und
Aktualisieren (210) des Erfassungsbereichs (42) auf der Grundlage der Benutzereingabe.

10. Verfahren nach einem der Ansprüche 1 bis 9, ferner umfassend:
Anweisen (212) des medizinischen Bilderfassungsgeräts (10), den Erfassungsbereich (24) abzubilden.

11. Verfahren nach Anspruch 10,
wobei das Anweisen (212) der medizinischen Bilderfassungsvorrichtung (10), den Erfassungsbereich (24) abzubilden, eine Bewegung einer Patientenliege (36) auslöst, die den Körper (28) des Patienten oder einen Teil davon trägt.

12. Verfahren nach Anspruch 10 oder 11, soweit abhängig von Anspruch 7,
wobei das Anweisen (212) der medizinischen Bilderfassungsvorrichtung (10), den Erfassungsbereich (24) abzubilden, ein Erfassen separater medizinischer Bilddaten für jede der Vielzahl von Unterbereichen (44; 46; 48) durch die medizinische Bilderfassungsvorrichtung (10) auslöst.

13. Verfahren nach einem der Ansprüche 1 bis 12,
wobei mindestens eine der einen oder mehreren Posen auf Grundlage von Planungsdaten erhalten wird, die die mindestens eine Pose relativ zu den zuvor aufgenommenen medizinischen Bilddaten des Teils des Körpers des Patienten (28) anzeigen.

14. Verfahren nach einem der Ansprüche 1 bis 13,
wobei mindestens eine der einen oder mehreren Posen auf der Grundlage von Tracking-Daten erhalten wird, die die mindestens eine Pose relativ zum Körper des Patienten (28) anzeigen.

15. Verfahren nach einem der Ansprüche 1 bis 14,
wobei jedes des mindestens einen anatomischen Elements (52; 56; 58; 64; 66; 68) ein Knochen ist, und
wobei das mindestens eine chirurgische Gerät (26; 34; 54; 60; 70) mindestens Gerät umfasst, das ausgewählt ist aus (i) einem chirurgischen Instrument (26; 34) und (ii) einem medizinischen Implantat (54; 60; 70).

16. Vorrichtung (2) umfassend mindestens einem Prozessor (4) und mindestens einen Speicher (6), wobei der mindestens eine Speicher (6) Befehle enthält, die, wenn sie von dem mindestens einen Prozessor (4) ausgeführt werden, den mindestens einen Prozessor dazu veranlassen:
für jedes von mindestens einem chirurgischen Gerät (26; 34; 54; 60; 70) Informationen zu erhalten (202), die eine oder mehrere Posen desselben anzeigen;
auf Grundlage der einen oder mehreren Posen mindestens ein anatomisches Element (52; 56; 58; 64; 66; 68) des Körpers eines Patienten auszuwählen (204); und
auf Grundlage des ausgewählten mindestens einen anatomischen Elements (52; 56; 58; 64; 66; 68) einen Erfassungsbereich (42) zu bestimmen (206), der von einer medizinischen Bilderfassungsvorrichtung (10) abzubilden ist,
wobei das mindestens eine anatomische Element (52; 56; 58; 64; 66; 68) auf Grundlage von zuvor aufgenommenen medizinischen Bilddaten von mindestens einem Teil des Körpers (28) des Patienten ausgewählt wird, wobei der Teil das mindestens eine anatomische Element (52; 56; 58; 64; 66; 68) umfasst.

17. Vorrichtung (2) nach Anspruch 16, derart konfiguriert, dass sie das Verfahren nach einem der Ansprüche 2 bis 15 durchführt.

18. Medizinisches Bildgebungssystem (100), umfassend die Vorrichtung (2) nach Anspruch 16 und die medizinische Bilderfassungsvorrichtung (10), sowie optional eine Patientenliege, die den Körper (28) des Patienten oder einen Teil davon trägt.

19. Computerprogrammprodukt umfassend Programmcodeabschnitte zur Durchführung des Verfahrens nach einem der Ansprüche 1 bis 15, wenn das Computerprogrammprodukt auf einem oder mehreren Prozessoren (4) ausgeführt wird.

## Revendications

1. Procédé de détermination d'une région de balayage (42) à imager par un dispositif d'acquisition d'images (10), le procédé étant exécuté par au moins un processeur (4) et comprenant:
l'obtention (202), pour chacun d'au moins un dispositif chirurgical (26; 34; 54; 60; 70), d'informations indiquant l'un ou plusieurs poses de celui-ci;
la sélection, sur la base d'une ou plusieurs poses, d'au moins un élément anatomique (52; 56; 58; 64; 66; 68) d'un corps de patient; et
la détermination, sur la base de l'au moins un élément anatomique sélectionné (52; 56; 58; 64; 66; 68), de la région de balayage (42) à imager par le dispositif d'acquisition d'images médicales (10),
dans lequel l'au moins un élément anatomique (52; 56; 58; 64; 66; 68) est sélectionné sur la base de données d'images médicales prises précédemment d'au moins une partie du corps de patient (28), la partie comprenant l'au moins un élément anatomique (52; 56; 58; 64; 66; 68).

2. Procédé selon la revendication 1,
dans lequel l'au moins un élément anatomique (52; 56; 58; 64; 66; 68) est sélectionné parmi un ou plusieurs éléments anatomiques (52; 56; 58; 64; 66; 68) identifiés dans les données d'images médicales prises précédemment.

3. Procédé selon la revendication 1 ou 2,
dans lequel chaque pose indique le dispositif chirurgical respectif (26; 34; 54; 60; 70) ayant une relation associée avec l'au moins un élément anatomique (52; 56; 58; 64; 66; 68) à sélectionner, par exemple, entrant en contact, s'étendant au moins partiellement dans ou étant aligné avec l'au moins un élément anatomique (52; 56; 58; 64; 66; 68) à sélectionner.

4. Procédé selon l'une quelconque des revendications 1 à 3,
dans lequel la région de balayage (42) est déterminée de sorte qu'il inclut ou exclut l'au moins un élément anatomique sélectionné (52; 56; 58; 64; 66; 68) ou de sorte qu'il inclut un premier ensemble de l'au moins un élément anatomique sélectionné (52; 56; 58; 64; 66; 68) et exclut un second ensemble de l'au moins un élément anatomique sélectionné (68).

5. Procédé selon la revendication 4,
dans lequel la région de balayage (42) est déterminée pour correspondre ou comprendre au moins un rectangle englobant virtuel (72, 74, 76) entourant un ou plusieurs de l'au moins un élément anatomique sélectionné (52; 56; 58; 64; 66).

6. Procédé selon l'une quelconque des revendications 1 à 5,
dans lequel la région de balayage (42) est déterminée pour satisfaire une ou plusieurs contraintes choisies parmi
(i) une amplitude de mouvement du dispositif d'acquisition d'images médicales (10),
(ii) une gamme d'imagerie spatiale du dispositif d'acquisition d'images médicales (10), et
(ii) une amplitude de mouvement d'un support de patient (36) supportant le corps du patient (28) ou une partie de ce dernier.

7. Procédé selon l'une quelconque des revendications 1 à 6,
dans lequel la région de balayage (42) est déterminée de sorte qu'elle comprenne une pluralité de sous-régions (44; 46; 48) à imager séparément par le dispositif d'acquisition d'images médicales (10).

8. Procédé selon l'une quelconque des revendications 1 à 7, comprenant en outre:
le déclenchement (206) de la visualisation d'une indication de la région de balayage (24).

9. Procédé selon l'une quelconque des revendications 1 à 8, comprenant:
l'obtention (208) l'entrée d'utilisateur indiquant un changement de la région de balayage (42); et
la mise à jour (210) de la région de balayage (42) sur la base de l'entrée d'utilisateur.

10. Procédé selon l'une quelconque des revendications 1 à 9, comprenant en outre:
l'ordre (212) au dispositif d'acquisition d'images médicales (10) d'imager la région de balayage (24).

11. Procédé selon la revendication 10,
dans lequel l'ordre (212) au dispositif d'acquisition d'images médicales (10) d'imager la région de balayage (24) déclenche le déplacement d'un support de patient (36) supportant le corps de patient (28) ou une partie de ce dernier.

12. Procédé selon la revendication 10 ou 11, pour autant qu'elle dépende de la revendication 7,
dans lequel l'ordre (212) au dispositif d'acquisition d'images médicales (10) d'imager la région de balayage (24) déclenche l'acquisition de données d'images médicales séparées pour chacune de la pluralité des sous-régions (44; 46; 48) par le dispositif d'acquisition d'images médicales (10).

13. Procédé selon l'une quelconque des revendications 1 à 12,
dans lequel au moins l'une parmi une ou plusieurs poses est obtenue sur la base de données de planification indiquant l'au moins une pose relative aux données d'images médicales prises précédemment de la partie du corps de patient (28).

14. Procédé selon l'une quelconque des revendications 1 à 13,
dans lequel au moins l'une parmi une ou plusieurs poses est obtenue sur la base de données de suivi de l'au moins une pose relative au corps de patient (28).

15. Procédé selon l'une quelconque des revendications 1 à 14,
dans lequel chacun de l'au moins un élément anatomique (52; 56; 58; 64; 66; 68) est un os, et
dans lequel l'au moins un dispositif chirurgical (26; 34; 54; 60; 70) comprend au moins un dispositif choisi parmi (i) un instrument chirurgical (26; 34) et (ii) un implant médical (54; 60; 70).

16. Appareil (2) comprenant au moins un processeur (4) et au moins une mémoire (6), l'au moins une mémoire (6) contenant des instructions qui, lorsqu'elles sont exécutées par l'au moins un processeur (4), amènent l'au moins un processeur à:
obtenir (202), pour chacun d'au moins un dispositif chirurgical (26; 34; 54; 60; 70), des informations indiquant l'un ou plusieurs poses de celui-ci;
sélectionner, sur la base d'une ou plusieurs poses, au moins un élément anatomique (52; 56; 58; 64; 66; 68) d'un corps de patient; et
déterminer, sur la base de l'au moins un élément anatomique sélectionné (52; 56; 58; 64; 66; 68), une région de balayage (42) à imager par le dispositif d'acquisition d'images médicales (10),
dans lequel l'au moins un élément anatomique (52; 56; 58; 64; 66; 68) est sélectionné sur la base de données d'images médicales prises précédemment d'au moins une partie du corps du patient (28), la partie comprenant l'au moins un élément anatomique (52; 56; 58; 64; 66; 68).

17. Appareil (2) selon la revendication 16, configuré pour exécuter le procédé selon l'une quelconque des revendications 2 à 15.

18. Système d'imagerie médical (100) comprenant l'appareil (2) selon la revendication 16 et le dispositif d'acquisition d'images médicales (10), et, éventuellement, un support de patient supportant le corps de patient (28) ou une partie de ce dernier.

19. Produit programme d'ordinateur comprenant des parties de code de programme pour exécuter le procédé selon l'une quelconque des revendications 1 et 15 lorsque le produit programme d'ordinateur est exécuté sur un ou plusieurs dispositifs (4).
